# EUROPÄISCHE PATENTSCHRIFT

(11) **EP 1 385 559 B1**
(45) Veröffentlichungstag und Bekanntmachung des Hinweises auf die Patenterteilung: **12.01.2005**
(21) Anmeldenummer: 02735229.3
(22) Anmeldetag: 05.04.2002
(51) Int. Cl.: A61L 27/20

(54) **PORÖSE UND NICHTPORÖSE MATRICES AUF BASIS VON CHITOSAN UND HYDROXYCARBONSÄUREN**
CHITOSAN AND HYDROXY CARBOXYLIC ACID BASED POROUS AND NON-POROUS MATRICES
MATRICES POREUSES ET NON POREUSES A BASE DE CHITOSANE ET D'ACIDES HYDROXYCARBOXYLIQUES

(30) Priorität: 06.04.2001 DE 10117234
(43) Veröffentlichungstag der Anmeldung: 04.02.2004
(73) Patentinhaber: Alvito Biotechnologie GmbH, 14532 Kleinmachnow (DE)
(72) Erfinder: PAHMEIER, Andrea, 15806 Zossen (DE); HAHNEMANN, Birger, 15806 Zossen (DE); SPERLING, Philipp, 14165 Berlin (DE); LÖTZBEYER, Thomas, 85386 Eching (DE)
(74) Vertreter: Weiss, Wolfgang, Dipl.-Chem. Dr.
(86) Internationale Anmeldenummer: PCT/EP2002/003798
(87) Internationale Veröffentlichungsnummer: WO 2002/080992

(56) Entgegenhaltungen:
- EP-A- 0 627 225
- EP-A- 0 704 222
- EP-A- 0 878 129
- WO-A-01/25321

## Beschreibung

Die Erfindung betrifft biokompatible Matrices auf Basis von Chitosan und Hydroxycarbonsäuren, diese Matrices enthaltende mehrschichtige Systeme und Anwendungen solcher Matrices.

Auf dem Gebiet der Transplantationsmedizin sind in den letzten Jahren erhebliche Erfolge erzielt worden. Probleme bereiten jedoch die geringen verfügbaren Mengen an Spenderorganen sowie durch heterologe Spenderorgane verursachte Abstoßungsreaktionen. Ein weiteres Problem besteht darin, dass mit heterologen Spenderorganen auch Krankheitserreger übertragen werden können. Es sind daher Versuche unternommen worden, künstliche Organe aus Zellkulturen auf einer dreidimensionalen Matrix zu züchten, die entsprechend den Bedürfnissen geformt werden kann, beispielsweise als Ohr. Dieses künstliche Organ oder Körperteil kann dann transplantiert werden, wobei bei Verwendung körpereigener Zellen keine Abstoßungsreaktion eintritt.

Als vielversprechendes Matrixmaterial hat Chitosan ein immer größeres Interesse gefunden. Chitosan ist ein teilweise deacetyliertes Chitin und wird aus den Exoskeletten von Arthropoden gewonnen. Es ist ein Aminopolysaccharid (Poly-1-4-glucosamin), das beispielsweise im Medizinbereich als Nahtmaterial oder zur Verkapselung von Pharmaka verwendet wird. Sein Vorteil liegt darin, dass es vom Körper vollständig resorbiert werden kann. Chitosan kann im leicht Sauren (pH < 6) durch Protonierung der freien Aminogruppen in Wasser gelöst werden. Im Alkalischen (pH > 7) fällt es aus der wässrigen Lösung wieder aus. Durch diesen pH-abhängigen Mechanismus, kann Chitosan unter milden Bedingungen gereinigt und verarbeitet werden.

In der US 5,871,985 wird ein Träger für die Transplantation in einen Patienten vorgeschlagen, der aus einer Matrix besteht, in die Zellen eingewachsen sind. Dazu wird zunächst eine Lösung aus Chitosan hergestellt, in der lebende Zellen enthalten sind. Diese Lösung wird dann in eine semipermeable Membran eingeschlossen, um den Träger auszubilden. Das Chitosan wird präzipitiert und bildet eine unvernetzte Matrix aus, in der die Zellen verteilt sind.

Madihally et al. (Biometerials 1999; 20(12), S. 1133-1142) beschreibt eine Matrix für die Gewebegeneration. Chitosan, das zu 85-90 % deacetyliert ist, wird dazu in 0,2 M Essigsäure gelöst, so dass Lösungen mit einem Gehalt von 1 bis 3 Gew.-% Chitosan erhalten werden. Die Lösung wird eingefroren und das Wasser und die überschüssige Essigsäure durch Lyophilisieren entfernt.

Die Deutsche Patentanmeldung 199 48 120.2 offenbart ein Verfahren zur Herstellung einer biokompatiblen dreidimensionalen Matrix, wobei eine wässrige Lösung eines Chitosans und einer im Überschuss vorliegenden Säure, insbesondere einer Hydroxycarbonsäure, eingefroren wird und das Wasser bei vermindertem Druck absublimiert wird, wobei die überschüssige Säure vor dem Einfrieren oder nach dem Absublimieren des Wassers entfernt, insbesondere neutralisiert wird. Weiterhin wird eine durch das Verfahren erhältliche Matrix offenbart, die zur Herstellung von Implantaten verwendet werden kann.

Ausgehend von dieser Erkenntnis war es die Aufgabe der vorliegenden Erfindung, neue Matrixformen oder/und Anwendungen einer Matrix auf Basis von Chitosan und einer Säure, insbesondere einer Hydroxycarbonsäure bereitzustellen.

Ein erster Aspekt der vorliegenden Erfindung betrifft daher eine biokompatible nichtporöse Matrix auf Basis von Chitosan und einer Säure, insbesondere einer Hydroxycarbonsäure, die beispielsweise in Form einer Folie oder eines dreidimensionalen Körpers, z.B. eines Hohlkörpers oder einer Rolle, sein kann. Die nichtporöse Matrix ist erhältlich durch:
- Bereitstellen einer wässrigen Lösung eines Chitosans und einer im Überschuss vorliegenden Säure, insbesondere einer Hydroxycarbonsäure,
- Trocknen der Lösung ohne Einfrieren und
- Entfernen von überschüssigen Säuren vor oder/und nach dem Trocknen, vorzugsweise durch Neutralisation.

Die nichtporöse Matrix kann als Träger für eine poröse dreidimensionale Matrix verwendet werden. Somit können biokompatible Matrixsysteme bereitgestellt werden, die mindestens eine biokompatible poröse Matrix, wie zuvor beschrieben, und mindestens eine biokompatible poröse Matrix umfassen. Die biokompatible poröse Matrix ist vorzugsweise auf Basis von Chitosan und einer Säure, insbesondere einer Hydroxycarbonsäure aufgebaut. Es können jedoch auch andere poröse biokompatible Matrices verwendet werden.

Besonders bevorzugt ist eine biokompatible poröse Matrix gemäß der Deutschen Anmeldung 199 48 120.2, die erhältlich ist durch:
- Bereitstellen einer wässrigen Lösung eines Chitosans und einer im Überschuss vorliegenden Säure, insbesondere einer Hydroxycarbonsäure,
- Einfrieren und Trocknen der Lösung, insbesondere durch Absublimieren bei verringertem Druck und
- Entfernen von überschüssiger Säure vor oder/und nach dem Einfrieren, insbesondere durch Neutralisation mit einer geeigneten Base, z.B. NaOH.

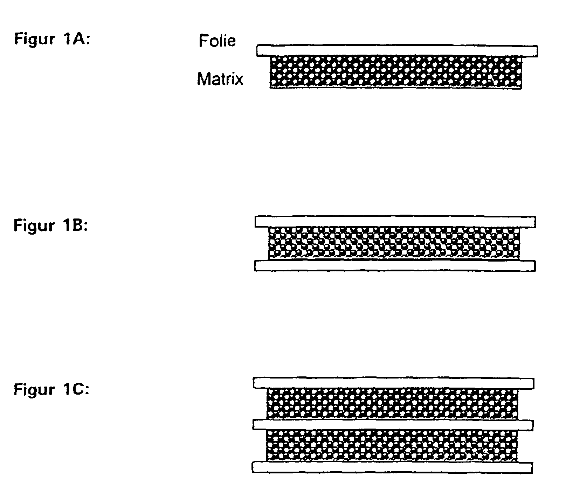

Bei erfindungsgemäßen Matrixsystem können nichtporöse Matrices und poröse Matrices jeweils abwechselnd in Schichten angeordnet sein. Beispiele für derartige Mehrschichtsysteme sind in Figur 1 A, 1 B und 1 C dargestellt. Alternativ kann eine nichtporöse Matrix auch zwischen zwei porösen Matrices angeordnet sein.

Die erfindungsgemäße nichtporöse Matrix oder das darauf basierende Matrixsystem kann zur in vitro Kultivierung von Zellen verwendet werden. Dabei kann das Matrixsystem zusätzliche Faktoren zum Zellwachstum, z.B. Cytokine, enthalten.

Beispielsweise können die Matrix oder das Matrixsystem eingesetzt werden zur Züchtung von Knorpelgewebe, zur Rekonstruktion von Knochengewebe, als Füllmaterial für Bioreaktoren zur Produktion von Zellen, Proteinen oder Viren, als Mikrocarrier von Füllmaterial für Bioreaktoren, zur Erzeugung von Kapillaren und Blutgefäßen, zur Erzeugung von gegebenenfalls mehrschichtigen Hautsystemen, zur Kultivierung von Blutstammzellen, zur Regeneration von Nervengeweben, zur Erzeugung künstlicher Organe.

Eine besonders bevorzugte Anwendung des mehrschichtigen Matrixsystems ist die Herstellung eines Basismaterials zur Erzeugung eines mehrschichtigen künstlichen Hautsystems. Dabei kann das Matrixsystem mit Keratinocyten sowie gegebenenfalls zusätzlich mit Fibroblasten besiedelt werden. Weiterhin kann ein vaskularisiertes Hautsystem erzeugt werden, wobei in die porösen Schichten des Matrixsystems Röhren eingezogen werden, die nach Besiedelung mit Epithelzellen zur Vaskularisierung der künstlichen Haut beitragen.

Eine weitere besonders bevorzugte Anwendung des mehrschichtigen Matrixsystems ist die Erzeugung einer künstlichen Herzklappe, wobei eine nichtporöse Struktur zur Erhöhung der mechanischen Stabilität zwischen zwei porösen Strukturen eingebaut und dann zur Kultivierung von Muskelzellen verwendet wird.

Weiterhin kann die nichtporöse Matrix und das darauf basierende Matrixsystem auch als Implantat ohne vorherige Zellbesiedelung, z.B. bei Knorpel- und Knochendefekten, zum Ersatz von Mikrokapillaren oder als chirurgisches Füllmaterial, z.B. für die rekonstruktive Chirurgie oder die Schönheitschirurgie eingesetzt werden.

Ein weiterer Aspekt der vorliegenden Erfindung betrifft eine biokompatible Matrix auf Basis von Chitosan und einer Säure, insbesondere einer Hydroxycarbonbsäure mit anisotropen Strukturen, beispielsweise parallel ausgerichteten Fasern oder/und Kammern. In dieser Ausführungsform ist die Matrix vorzugsweise porös. Die anisotrope Matrix ist erhältlich durch:
- Bereitstellen einer wässrigen Lösung eines Chitosans und einer im Überschuss vorliegenden Säure, insbesondere einer Hydroxycarbonsäure,
- anisotropes Einfrieren und Trocknen der Lösung, insbesondere durch Absublimation bei verringertem Druck und
- Entfernen von überschüssiger Säure vor oder/und nach dem Einfrieren.

Das anisotrope Einfrieren umfasst vorzugsweise ein Einfrieren unter Verwendung von strukturierten Kälteelementen, z.B. Rohren in direktem oder indirektem Kontakt mit der Matrix während des Einfrierprozesses. Die Kältelemente können langgestreckt sein, um beispielsweise parallel ausgerichtete Fasern oder Kammern in der Matrix zu erhalten. Es können jedoch auch gekrümmte Strukturen, z.B. Nachbildungen des zu formenden Organs, als Kälteelemente verwendet werden.

Die anisotrope poröse Matrix kann in einem biokompatiblen Matrixsystem zusammen mit einer anderen Matrix, beispielsweise mit einer biokompatiblen nichtporösen Matrix eingesetzt werden. Die anisotrope Matrix bzw. das darauf basierende Matrixsystem kann zur in vitro Kultivierung von Zellen oder als Implantat ohne vorherige Zellbesiedlung, entsprechend den zuvor genannten Anwendungen, eingesetzt werden.

Noch ein weiterer Aspekt der Erfindung ist die Verwendung einer biokompatiblen Matrix auf Basis von Chitosan und einer Säure, insbesondere einer Hydroxycarbonsäure, wie in DE 199 48 120.2 beschrieben, zur Züchtung von Knorpelgewebe, zur Rekonstruktion von Knochengewebe, als Füllmaterial für Bioreaktoren zur Produktion von Zellen, Proteinen oder Viren, als Mikrocarrier von Füllmaterial für Bioreaktoren, zur Erzeugung von Kapillaren und Blutgefäßen, zur Erzeugung von gegebenenfalls mehrschichtigen Hautsystemen, zur Kultivierung von Blutstammzellen, zur Regeneration von Nervengeweben, zur Erzeugung künstlicher Organe.

Überraschenderweise wurde festgestellt, dass Zellen in einer Dichte 10⁶ oder mehr Zellen pro cm² Matrix kultiviert werden können. Dabei handelt es sich um eine im Vergleich zur Kultivierung in einer Kulturschale mehr als 10fache Erhöhung der Zelldichte.

Die Herstellung der erfindungsgemäßen Matrices auf Basis von Chitosan und Säuren erfolgt im Wesentlichen nach dem in der Deutschen Anmeldung 199 48 120.2 angegebenen Verfahren, sofern nichts anderes angegeben ist. Vorzugsweise wird zunächst eine wässrige Lösung eines teilweise deacetylierten Chitosans und einer im Überschuss vorliegenden Säure hergestellt. Unter Überschuss wird dabei verstanden, dass der pH der wässrigen Lösung im Sauren liegt, vorzugsweise unterhalb von pH ≤ 4. Dadurch sind die freien Aminogruppen des Chitosans zumindest teilweise protoniert, wodurch die Löslichkeit in Wasser gesteigert wird. Die Säuremenge ist nicht kritisch. Sie muss lediglich so gewählt sein, dass das Chitosan in Lösung geht. Eine übermäßige Säurezugabe wird nach Möglichkeit vermieden, da überschüssige Säure wieder entfernt werden muss, und dadurch die Aufarbeitung bei großen Säuremengen erschwert wird. Günstig sind Säuremengen, die eine 0,05 bis 1 N, vorzugsweise 0,1 bis 0,5 N, insbesondere 0,1 bis 0,3 N Lösung ergeben. Die Chitosanmenge wird vorzugsweise so gewählt, dass sich eine 0,01 bis 0,5 M, vorzugsweise 0,1 bis 0,3 M Lösung ergibt. Durch die Konzentration der Chitosanlösung kann Einfluss auf die Struktur der Matrix, insbesondere deren Porengröße genommen werden. Auf diese Weise lässt sich die Porengröße der Matrix auf den jeweiligen Zelltypus abstimmen, mit dem die Matrix besiedelt werden soll.

Chitosan hat wegen seiner Herstellung aus natürlichen Quellen kein einheitliches Molekulargewicht. Je nach Quelle und Aufbereitungsverfahren kann das Molekulargewicht zwischen 20 kDa bis über 1000 kDa betragen.

Für die Herstellung der dreidimensionalen Matrix ist das Chitosan hinsichtlch seines Molekulargewichts keinen Beschränkungen unterworfen. Für die Herstellung der wässrigen Chitosanlösung wird eine Säure verwendet, bei der es sich um eine anorganische Säure oder vorzugsweise um eine organische Säure, besonders bevorzugt um eine Alkyl- oder Aryl-Hydroxycarbonsäure handelt. Geeignet sind insbesondere Hydroxycarbonsäuren mit 2 bis 12 Kohlenstoffatomen, wobei eine oder mehrere Hydroxylgruppen sowie eine oder mehrere Carboxylgruppen im Molekül vorhanden sein können. Spezifische Beispiele sind Glycolsäure, Milchsäure, Äpfelsäure, Weinsäure, Zitronensäure und Mandelsäure. Besonders bevorzugt ist Milchsäure.

Bei Herstellung einer porösen Matrix wird die Lösung aus Chitosan und Säure zunächst durch Zugabe von Base zumindest teilweise neutralisiert und dann eingefroren oder ohne vorherige Neutralisation direkt eingefroren. Die Neutralisation vor dem Einfrieren ist bevorzugt. Der pH-Wert nach der Neutralisation beträgt im Allgemeinen 5,0 bis 7,5, vorzugsweise 5,5 bis 7,0 und insbesondere 6,0 bis 7,0.

Nach dem Einfrieren wird das Wasser unter vermindertem Druck absublimiert, beispielsweise im Druckbereich von 0,001 bis 3 hPa.

Zur Herstellung einer nichtporösen Matrix wird die Lösung nicht gefroren und absublimiert, sondern ohne Einfrieren bei gegebenenfalls erhöhter Temperatur oder/und verringertem Druck getrocknet und vorzugsweise nach Trocknung neutralisiert. Die entstehende nichtporöse Matrix ist in feuchtem Zustand stark belastbar und dehnbar.

Durch die große Anzahl von Amino- und Hydroxygruppen ist die Matrix beliebig modifizierbar. Bei einer bevorzugten Ausführungsform der dreidimensionalen Matrix sind Liganden kovalent oder nicht kovalent an die Chitosanmatrix gebunden, vorzugsweise an die freien Aminogruppen des Chitosans. Als Liganden können z.B. Wachstumsstoffe, Proteine, Hormone, Heparin, Heparansulfate, Chondroitsulfate, Dextransulfate oder eine Mischung dieser Substanzen verwendet werden. Die Liganden dienen vorzugsweise zur Kontrolle und Verbesserung der Zellproliferation.

In einer bevorzugten Ausführungsform der Erfindung werden Nukleinsäuren, z.B. RNA oder DNA, als Liganden in der Matrix verwendet. Die Nukleinsäuren können über chemische Kopplung an die im Chitosan vorhandenen Amino- oder/und Hydroxygruppen immobilisiert werden. Mit einer Nukleinsäure-beladenen Matrix kann eine lokal begrenzte transiente Expression heterologer Gene im Körper erreicht werden. Wird eine derart gekoppelte Matrix nämlich im Körper implantiert und von körpereigenen Zellen besiedelt, die die Matrix auflösen, nehmen die Zellen auch die darauf immobilisierten Nukleinsäuren auf und sind in der Lage, diese zu exprimieren.

Das Zellwachstum auf der Matrix wird weiter verbessert, wenn die Matrix mit autologem Fibrin beschichtet ist.

Die erfindungsgemäße dreidimentsionale Matrix kann als Festphase in einem Kulturreaktor (Cell Factory) verwendet werden. Die Matrix zeigt eine sehr hohe Beständigkeit im Kulturmedium. Ferner hat sich gezeigt, dass die Matrix das Zellwachstum fördert.

Die Matrix eignet sich ferner zur Verwendung als Zellimplantat, insbesondere für knorpelbilderide Zellen. Es müssen dabei keine gentechnisch veränderten Zellen verwendet werden. Die Zellen werden bevorzugt durch Biopsie dem Patienten entnommen, auf der Zellmatrix angezüchtet und das Zellimplantat dann dem Patienten eingepflanzt. Durch die Besiedlung der dreidimensionalen Matrix mit körpereigenen Stammzellen (Knochenersatz), die sich erst am Ort des Transplantats differenzieren, angeregt durch die jeweiligen Wachstumsfaktoren des umliegenden Gewebes, oder mit Knorpelzellen zur erneuten Bildung hyalinen Knorpels, sind Abstoßungsreaktionen des Transplantats weitgehend ausgeschlossen.

Die dreidimensionale Matrix kann sowohl mit humanen als auch mit animalischen Zellen (beispielsweise vom Pferd, Hund oder Hai) besiedelt werden. Besonders geeignet sind Haizellen, da diese beim Empfänger keine wesentliche immunologische Antwort auslösen. Haizellen werden bereits als Organersatz verwendet, z.B. für Augenlinsen. Beispiele für Zellen, mit denen die erfindungsgemäßen Matrices bzw. Matrixsysteme besiedelt werden können, sind Chondrocyten, Osteocyten, Keratinocyten, Hepatocyten, Knochenmarksstammzellen oder neuronale Zellen.

Die Matrices oder Matrxsysteme, wie zuvor beschrieben, können im humanmedizinischen und im tiermedizinischen Bereich eingesetzt werden. Weitere Einsatzgebiete sind die Verwendung als Einmalartikel als in vitro Testsystem zur Untersuchung von pharmazeutischen Wirkstoffen. Hierzu können beispielsweise Blutstammzellen oder Hepatocyten auf der Matrix kultiviert werden. Dieses System kann zur Untersuchung der Wirksamkeit von Testsubstanzen aus einer chemischen oder/und biologischen Substanzenbibliothek; gegebenenfalls in einem Hochdurchsatzverfahren, verwendet werden.

Die Matrix und das Matrixsystem werden vor Einsatz in der Zellkultur sterilisiert, um Keimfreiheit zu garantieren. Die Sterilisation kann durch Temperaturbehandlung, z.B. durch Autoklavieren, Dampfbehandlung etc. oder/und durch Bestrahlung, z.B. Gammastrahlenbehandlung, erfolgen. Vorzugsweise erfolgt die Sterilisation in einer physiologisch verträglichen gepufferten Lösung, z.B. in PBS, um eine vollständige Benetzung der Matrix mit Flüssigkeit und die Abwesenheit größerer Lufteinschlüsse sicherzustellen.

Bei Kultivierung der Zellen wird die Matrix in einem Zeitraum von ca. 5-8 Wochen abgebaut. Die Abbauzeiten können über den Deacetylierungsgrad des Chitosans und die Konzentration des Materials eingestellt werden.

Weiterhin soll die Erfindung durch die nachfolgenden Beispiele erläutert werden.

### Beispiel 1: Herstellung einer nichtporösen Folie

Nach dem im Beispiel 3 von DE 199 48 120.2 beschriebenen Verfahren wird eine Mischung aus Chitosan und Milchsäure hergestellt. Die Lösung wird in eine Petrischale gegossen, bei 50 °C getrocknet und nach Entstehen eines glasklaren Filmes mit 1 M Natronlauge auf einen pH-Wert von 7 neutralisiert. Die entstehende Folie ist im feuchten Zustand stark belastbar und dehnbar.

### Beispiel 2: Wachstum von Hep-G2-Zellen in der Matrix

Zwei definierte Anfangsmengen, 1 x 10⁵ bzw. 1 x 10⁶, Hep-G2-Hepatocyten wurden in ein 1, 5 cm² großes Stück poröse Matrix (hergestellt nach Beispiel 3 von DE 199 48 120.2) eingespritzt und das Zellwachstum zu vier Zeitpunkten, maximal 33 Tage lang beobachtet. Dabei konnte ein kontinuierliches Zellwachstum beobachtet werden.

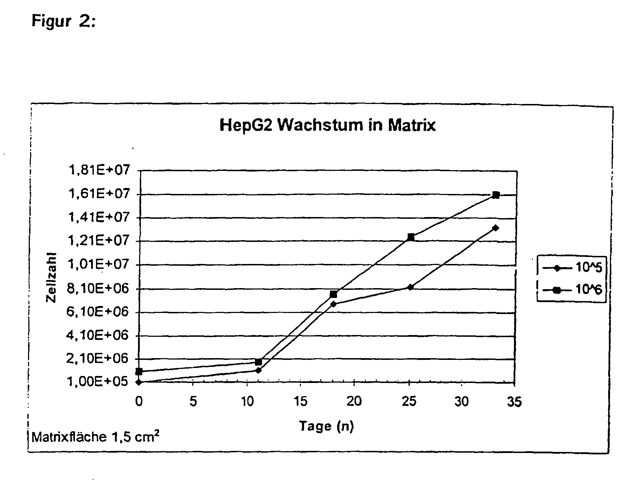

Nach 33 Tagen lag die maximale Zellzahl pro Matrix bei 1,6 x 10⁷ Zellen (Figur 2). Das bedeutet, dass sich die Zellzahl auf der geringen Grundfläche von 1,5 cm² noch um eine Zehnerpotenz vermehren konnte. Die Zelldichte einer konfluenten, konventionellen Kulturschale mit einer Grundfläche von 80 cm² wird beim der Deutschen Sammlung von Mikroorganismen und Zellkulturen (DSMZ) mit 2,5-3,0 x 10⁷ für Hep-G2 angegeben. Diese Menge ist, wenn die auf die Grundfläche der Matrix umgelegt wird, etwa 25fach geringer als die Zellzahl, die nach 33 Tagen in der Matrix nachgewiesen werden konnte.

### Beispiel 3: Einfluss der Matrix auf die Zellproliferation

Bei diesem Versuch sollte gezeigt werden, ob sich Substanzen, die sich in der Matrix befinden, das Zellwachstum ungünstig beeinflussen. Dabei sollte nicht das Wachstum der Zellen auf der Matrix begutachtet werden, sondern nur der Einfluss potenzieller, löslicher Substanzen, die möglicherweise an das Medium abgegeben werden. Dazu wurde 6 Tage lang ein 1,5 cm² großes Stück einer Matrix (hergestellt nach Beispiel 3 von DE 199 48 120.2) in 3 ml Zellkulturmedium bei 37 °C und 5 % CO₂ vorinkubiert. Das Medium wurde anschließend zusammen mit ebenfalls vorinkubierten Kontrollmedien in einem Standard-Proliferationstest (XTT) analysiert. Bei diesem Test werden durch metabolisch aktive Zellen ein Tetrazoliumsalz in ein farbiges Formazansalz umgesetzt, welches anschließend photometrisch nachgewiesen werden kann. Dabei konnte keine Beeinflussung des Zellwachstums beobachtet werden. Als Zelllinie wurde Hep-G2 verwendet und als Positivkontrolle wurde dem Medium 5 % DMSO zugesetzt. Der Test wurde dreifach wiederholt und lieferte in allen drei Fällen das gleiche Ergebnis.

### Beispiel 4: Wachstum anderer Zelllinien in der Matrix und Zellmorphologie

Neben Hep-G2 wurden zwei weitere Zelllinien auf die Matrix ausgesät, um zu beobachten, ob sie in der Matrix wachsen. Sowohl Hela als auch die Zelllinie CHO-K1 lässt sich in der Matrix vermehren.

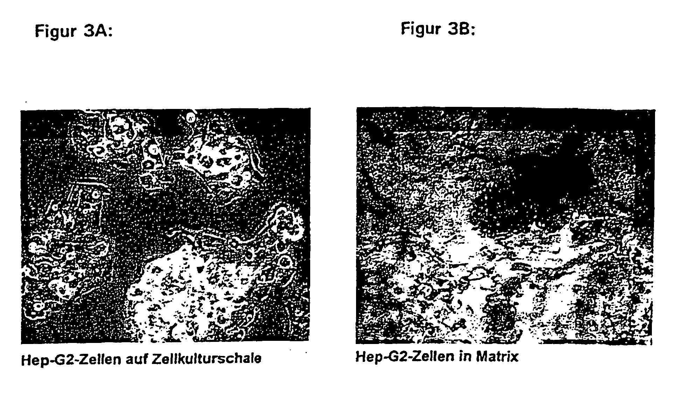

Bei allen drei Zelllinien lässt sich eine veränderte Morphologie im Vergleich zu Zellen, die in normalen Zellkulturschalen wachsen, beobachten. Die Zellen sind deutlich abgerundet und wachsen auch in die dritte Dimension und ähneln somit mehr Zellen in natürlichen dreidimensionalen Geweben. Als Beispiel sind in Figur 3 zwei Abbildungen der Leberzelllinie Hep-G2 dargestellt, wobei Figur 3A die Zellen nach Kultivierung aus einer Zellkulturschale und Figur 3B die Zellen nach Kultivierung in einer Matrix zeigt.

## Patentansprüche

1. Biokompatible nichtporöse Matrix auf Basis von Chitosan und einer Säure, insbesondere einer Hydroxycarbonsäure, erhältlich durch:
- Bereitstellen einer wässrigen Lösung eines Chitosans und einer im Überschuss vorliegenden Säure, insbesondere einer Hydroxycarbonsäure,
- Trocknen der Lösung ohne Einfrieren und
- Entfernen von überschüssigen Säuren vor oder/und nach dem Trocknen.

2. Biokompatibles Matrixsystem umfassend mindestens eine biokompatible nichtporöse Matrix nach Anspruch 1 und mindestens eine biokompatible poröse Matrix.

3. Matrixsystem nach Anspruch 2,
**dadurch gekennzeichnet,**
**dass** mindestens eine biokompatible poröse Matrix auf Basis von Chitosan und einer Säure, insbesondere einer Hydroxycarbonsäure aufgebaut ist.

4. Verwendung einer nichtporösen Matrix nach Anspruch 1 oder eines Matrixsystems nach einem der Ansprüche 2 bis 3 zur in vitro Kultivierung von Zellen, insbesondere zur Züchtung von Knorpelgewebe, zur Rekonstruktion von Knochengewebe, als Füllmaterial für Bioreaktoren zur Produktion von Zellen, Proteinen oder Viren, als Mikrocarrier von Füllmaterial für Bioreaktoren, zur Erzeugung von Kapillaren und Blutgefäßen, zur Erzeugung von gegebenenfalls mehrschichtigen Hautsystemen, zur Kultivierung von Blutstammzellen, zur Regeneration von Nervengeweben und zur Erzeugung künstlicher Organe.

5. Verwendung einer nichtporösen Matrix nach Anspruch 1 oder eines Matrixsystems und einem der Ansprüche 3 bis 3 als Implantat ohne vorherige Zellbesiedlung, insbesondere bei Knorpel- und Knochendefekten, als Mikrokapillaren oder als chirurgisches Füllmaterial.

6. Biokompatible Matrix auf Basis von Chitosan und einer Säure, insbesondere einer Hydroxycarbonsäure mit anisotropen Strukturen.

7. Biokompatibles Matrixsystem umfassend mindestens eine biokompatible anisotrope poröse Matrix nach Anspruch 6 und mindestens eine biokompatible nichtporöse Matrix.

8. Verwendung einer anisotropen Matrix nach Anspruch 6 oder eines Matrixsystems nach Anspruch 7 zur in vitro Kultivierung von Zellen oder als Implantat ohne vorherige Zellbesiedelung.

9. Biokompatible Matrix auf Basis von Chitosan und einer Säure, insbesondere einer Hydroxycarbonsäure,
**dadurch gekennzeichnet,**
**dass** sie Nukleinsäuren in chemisch angekoppelter Form enthält.

10. Verwendung einer biokompatiblen Matrix auf Basis von Chitosan und einer Säure, insbesondere einer Hydroxycarbonsäure, zur Züchtung von Knorpelgewebe, zur Rekonstruktion von Knochengewebe, als Füllmaterial für Bioreaktoren zur Produktion von Zellen, Proteinen oder Viren, als Mikrocarrier von Füllmaterial für Bioreaktoren, zur Erzeugung von Kapillaren und Blutgefäßen, zur Erzeugung von gegebenenfalls mehrschichtigen Hautsystemen, zur Kultivierung von Blutstammzellen, zur Regeneration von Nervengeweben und zur Erzeugung künstlicher Organe.

## Claims

1. Biocompatible non-porous matrix based on chitosan and an acid, in particular a hydroxy carboxylic acid, obtainable by:
- providing an aqueous solution of a chitosan and of an acid, in particular a hydroxy carboxylic acid, which is present in excess,
- drying the solution without freezing and
- removing excess acids before or/and after the drying.

2. Biocompatible matrix system comprising at least one biocompatible non-porous matrix according to Claim 1 and at least one biocompatible porous matrix.

3. Matrix system according to Claim 2, **characterized in that** at least one biocompatible porous matrix has a structure based on chitosan and an acid, in particular a hydroxy carboxylic acid.

4. Use of a non-porous matrix according to Claim 1 or of a matrix system according to any of Claims 2 to 3 for the in vitro culturing of cells, in particular for culturing cartilage tissue, for reconstructing bone tissue, as filling material for bioreactors for producing cells, proteins or viruses, as microcarrier of filling material for bioreactors, for generating capillaries and blood vessels, for generating optionally multilayer skin systems, for culturing blood stem cells, for regenerating nerve tissue and for generating artificial organs.

5. Use of a non-porous matrix according to Claim 1 or of a matrix system according to Claim 2 or Claim 3 as implant without previous cell colonization, in particular for cartilage and bone defects, as microcapillaries or as surgical filling material.

6. Biocompatible matrix based on chitosan and an acid, in particular a hydroxy carboxylic acid with anisotropic structures.

7. Biocompatible matrix system comprising at least one biocompatible anisotropic porous matrix according to Claim 6 and at least one biocompatible non-porous matrix.

8. Use of an anisotropic matrix according to Claim 6 or of a matrix system according to Claim 7 for the in vitro culturing of cells or as implant without previous cell colonization.

9. Biocompatible matrix based on chitosan and an acid, in particular a hydroxy carboxylic acid, **characterized in that** it comprises nucleic acids in chemically coupled-on form.

10. Use of a biocompatible matrix based on chitosan and an acid, in particular a hydroxy carboxylic acid, for culturing cartilage tissue, for reconstructing bone tissue, as filling material for bioreactors for producing cells, proteins or viruses, as microcarrier of filling material for bioreactors, for generating capillaries and blood vessels, for generating optionally multilayer skin systems, for culturing blood stem cells, for regenerating nerve tissue and for generating artificial organs.

## Revendications

1. Matrice non poreuse biocompatible à base de chitosane et d'un acide, en particulier d'un acide hydroxycarboxylique, pouvant être obtenue par :
- préparation d'une solution aqueuse d'un chitosane et d'un acide présent en excès, en particulier d'un acide hydroxycarboxylique,
- séchage de la solution sans congélation, et
- élimination des acides en excès avant ou/et après le séchage.

2. Système de matrices biocompatibles comprenant au moins une matrice non poreuse biocompatible selon la revendication 1 et au moins une matrice poreuse biocompatible.

3. Système de matrices selon la revendication 2, **caractérisé en ce que** l'on réalise au moins une matrice poreuse biocompatible à base de chitosane et d'un acide, en particulier d'un acide hydroxycarboxylique.

4. Utilisation d'une matrice non poreuse selon la revendication 1 ou d'un système de matrices selon l'une des revendications 2 à 3 pour cultiver des cellules *in vitro,* en particulier pour la culture de tissu cartilagineux, pour la reconstruction de tissu osseux, comme matériau de remplissage de bioréacteurs pour la production de cellules, de protéines ou de virus, comme microsupports de matériau de remplissage de bioréacteurs, pour la génération de capillaires et de vaisseaux sanguins, pour la génération de systèmes de peau le cas échéant multicouches, pour la mise en culture de cellules souches sanguines, pour la régénération de tissus nerveux et pour la génération d'organes artificiels.

5. Utilisation d'une matrice non poreuse selon la revendication 1 ou d'un système de matrices selon l'une des revendications 2 à 3 comme implant sans colonisation cellulaire préalable, en particulier dans le cas de défauts osseux et cartilagineux, comme microcapillaires ou comme matériau de remplissage chirurgical.

6. Matrice biocompatible à base de chitosane et d'un acide, en particulier d'un acide hydroxycarboxylique, ayant des structures anisotropes.

7. Système de matrices biocompatibles comprenant au moins une matrice poreuse anisotrope biocompatible selon la revendication 6 et au moins une matrice non poreuse biocompatible.

8. Utilisation d'une matrice anisotrope selon la revendication 6 ou d'un système de matrices selon la revendication 7 pour cultiver des cellules *in vitro* ou comme implant sans colonisation cellulaire préalable.

9. Matrice biocompatible à base de chitosane et d'un acide, en particulier d'un acide hydroxycarboxylique, **caractérisée en ce qu'**elle contient des acides nucléiques sous une forme couplée chimiquement.

10. Utilisation d'une matrice biocompatible à base de chitosane et d'un acide, en particulier d'un acide hydroxycarboxylique, pour la culture de tissu cartilagineux, pour la reconstruction de tissu osseux, comme matériau de remplissage de bioréacteurs pour la production de cellules, de protéines ou de virus, comme microsupports de matériau de remplissage de bioréacteurs, pour la génération de capillaires et de vaisseaux sanguins, pour la génération de systèmes de peau le cas échéant multicouches, pour la mise en culture de cellules souches sanguines, pour la régénération de tissus nerveux et pour la génération d'organes artificiels.
